# EUROPEAN PATENT SPECIFICATION

(11) **EP 1 157 094 B1**
(45) Date of publication and mention of the grant of the patent: **19.09.2007**
(21) Application number: 00908787.5
(22) Date of filing: 25.02.2000
(51) Int. Cl.: C12N 1/10, C12N 3/00

(54) **METHOD FOR THE PURIFICATION, RECOVERY, AND SPORULATION OF CYSTS AND OOCYSTS**
METHODE FÜR DIE REINIGUNG, GEWINNUNG UND SPORULATION VON ZYSTEN UND OOZYSTEN
METHODE DE PURIFICATION, RECUPERATION, ET SPORULATION DE KYSTES ET D'OOCYSTES

(30) Priority: 26.02.1999 US 122160 P
(43) Date of publication of application: 28.11.2001
(60) Divisional application: 07014547.9
(73) Proprietor: PFIZER INC., New York, N.Y. 10017-5755 (US)
(72) Inventor: CONKLE, Harold, N., Columbus, OH 43220 (US); BLONIGEN, Scott, J., Hilliard, OH 43026 (US); WERNER, Timothy, M., Columbus, OH 43212 (US); SHULTZ, Joseph, E., Camarillo, CA 93012 (US); KILANOWSKI, David, R., Dublin, OH 43017 (US); TEWKSBURY, Ted, L., Columbus, OH 43228 (US); MONZYK, Bruce, Delaware, OH 43015 (US); CUCKSEY, Chad, M., Columbus, OH 43220 (US); WEBER, Fred, H., Terra Haute, IN 47803 (US); MCARTHUR, Hamish, A., I., Gales Ferry, CT 06335 (US)
(74) Representative: Ebner von Eschenbach, Jennifer
(86) International application number: PCT/US2000/004733
(87) International publication number: WO 2000/050072

(56) References cited:
- US-A- 3 617 529
- US-A- 4 544 548
- US-A- 4 863 731
- US-A- 5 068 104
- RYLEY J F ET AL: "METHODS IN COCCIDIOSIS RESEARCH SEPARATION OF OOCYSTS FROM FECES" PARASITOLOGY, vol. 73, no. 3, 1976, pages 311-326, XP000909784 ISSN: 0031-1820
- SMITH P H ET AL: "FROTH FLOTATION FOR HARVESTING CHLORELLA ALGAE" NORTHWEST SCIENCE,US,PULLMAN, WA, vol. 42, no. 4, 1968, pages 165-171, XP002064294 ISSN: 0029-344X

## Description

### Background of the Invention

This invention relates generally to methods for the purification, recovery, and sporulation of encysted protozoa for use in the production of vaccines. Protozoa are pathogens known to attack the gastrointestinal tract of the host. In a situation where the host has a weak or suppressed immune system, such as very young, very old, and immuno-compromised hosts, infection may be fatal. Such a loss can translate into an economic loss as well. Protozoa are present in the environment in a relatively stable cyst form, also termed an oocyst when the protozoa is a sporozoa. Upon ingestion into the host, the encysted protozoa responds to the conditions of the gastrointestinal tract and infects the host.

In order to prevent or alleviate the problem of these diseases, non-in-ovo vaccines have been developed. These vaccines typically expose the host to a low level of the protozoa in order to develop immunity in the host without causing disease. Such vaccines include a vaccine against avian coccidiosis caused from any one of a number of species of coccidia (U.S. Patent No. 5,055,292), a vaccine for cats against toxoplasma (U.S. Patent No. 5,045, 313), and vaccines for pigs and other ruminants (U.S. Patent No. 4,808,404). These vaccines are only partially effective because they must be given by gavage or applied to the water or the food of the animal. Gavage is ineffective because it requires manual vaccine introduction. Also, many chicks die due to handling. Adding the vaccine to the food or water is also ineffective as young chicks eat little directly after hatching; therefore, disease control is delayed. The result is less rapid weight gain and a longer time to reach market weight.

In order to produce the vaccine, a supply of adequately purified encysted protozoa must be obtained. Current separation and recovery techniques employ corrosive, hazardous, toxic materials. Measures must be undertaken to compensate for these materials such as extensive washing operations to remove these materials which results in low product yield. The typical method of obtaining the encysted protozoa includes obtaining a source of encysted protozoa, such as the intestines or feces of infected animals. The intestinal matter or fecal matter needs to be separated from the encysted protozoa, and a flotation method is used. Heavy media flotation or sedimentation processes have been used where the heavy media is sodium chloride (U.S. Patent No. 4,863,731) or sucrose (U.S. Patent No. 5,068,104). The heavy media is used in an aqueous mixture. Unfortunately, the use of aqueous mixtures of the heavy media can have adverse effects. For example, aqueous mixtures of sodium chloride can lead to severe equipment corrosion.

Before the encysted sporozoa can become infective, the separated oocysts must be sporulated by mild oxidation. Mild oxidation is accomplished by either placing the suspension on a shaking table or bubbling air through the suspension of the oocysts. Potassium dichromate is typically employed to suppress any unwanted microbial growth during sporulation. But, potassium dichromate is a hazardous material. Its use creates handling and disposal problems. Its removal results in reduced yield and increased costs.

A final bleaching step using sodium hypochlorite is used to eliminate any remaining organic material and undesired microorganisms. The bleached oocysts must be washed to reduce the residual bleaching agent concentration to an acceptable level. Washing is accomplished by a series of dilutions and oocyst recovery operations to reduce the bleaching agent levels while producing an oocyst concentrate. The final oocyst concentrate is made into a vaccine under sterile conditions. Each vaccine may include multiple species of protozoa to provide the greatest amount of protection possible.

A need exists for a more efficient vaccination method. Such a vaccination method would employ a live vaccine dosed at a level sufficient to generate an immuno-response to build immunity but low enough not to cause acute symptoms. For example, an in-ovo vaccine against avian coccidiosis can shorten treatment times and allow a more uniform dosage. This method could also be used to collect and produce human vaccines for protection against protozoa including *Cryptosporidium* and *Giardia lamblia.* The method would employ a non-corrosive dense media flotation process or a gas flotation process that eliminates the need for salt entirely. This improved method would not use a hazardous biocide such as potassium dichromate. Such an alternative biocide or oxidant could also double as a bleaching agent, eliminating another step in the process and another hazardous compound.

### Summary of the Invention

The present invention is directed to a method for the sporulation of oocysts as recited in claim 1. In preferred embodiments the method of the invention is directed to the purification and recovery of the oocysts, including separating these from a suspension containing them by either a salt flotation process where the salt is salts such as sodium sulfate, magnesium sulfate, magnesium chloride, calcium chloride, or mixtures thereof, or a gas flotation process, prior to their sporulation.

In one embodiment, the method of separating the oocysts is accomplished by a salt flotation process and the salt is sodium sulfate. In this embodiment, the method includes preparing an admixture comprising the oocysts and the sodium sulfate centrifuging the slurry and recovering a supernatant therefrom, forming a dilution of the supernatant and centrifuging the dilution, and recovering the concentrate from the centrifuged dilution. This embodiment may also include homogenizing the admixture by high intensity homogenization. The sodium sulfate is present in the admixture in an amount from about 3 to about 30 weight percent. Further, the specific gravity of the dilution may be less than the specific gravity of the oocysts.

The concentrate comprises from about 1x10⁴ to about 1.5x10⁶ oocysts/ml.

In another embodiment, separation of the oocysts is accomplished by the gas flotation process. The gas flotation process includes adjusting the suspension to a pH sufficient to affect adhesion between bubbles of the gas in the suspension and the oocysts, conditioning the pH adjusted suspension by adding a sufficient amount of a surface active agent compound to selectively coat particles in the suspension and a sufficient amount of a heteropolar compound to produce a stable froth, passing the conditioned suspension through at least one gas flotation cell, and recovering the oocysts from the gas flotation cell. Suitable gases include air. When the gas is air, the pH can be adjusted to about 2.5 to about 3.5. The surface active agent compounds include a sodium salt of long-chain alkyl hydrogen sulfate, a quaternary ammonium compound, a blend of a fatty ammonium acetate and 2-ethylhexanol, an ester/amide compound, an alkyloxy polyethylenoxyethanol, and mixtures thereof. The heteropolar compounds include amyl alcohols, butyl alcohols, terpinols, cresols, and mixtures thereof. Suitable gas flow rates for the gas flotation cell range from about 0.25 to about 1.1 volumes of gas per volume of suspension per minute. In a preferred embodiment, the gas flotation cell comprises at least two serial gas flotation units, and these units can have different gas flow rates.

The method for the sporulation of oocysts of the present invention includes forming an aqueous suspension of the oocysts with water and hydrogen peroxide, wherein the hydrogen peroxide is present in an amount sufficient to eliminate unwanted microbiological growth, and aerating the aqueous suspension to sporulate the oocysts. The aqueous suspension may be aerated for a time period greater than about 40 hours under conditions such that the aqueous suspension during aeration has a dissolved oxygen level greater than about 80% of the saturation level at a temperature of about 22°C to about 32°C and may have an agitation level sufficient to adequately suspend all the solids. In this embodiment, the aqueous suspension can include an oocyst concentration of about 10⁴ to about 10⁶ oocysts/ml and a hydrogen peroxide concentration of about 1,000 to about 20,000 mg/l.

The present invention covers a method for the purification, recovery, and sporulation of oocysts including separating the oocysts from a first suspension comprising the oocysts, and sporulating the separated oocysts by the methods of the present invention. Suitable oocysts are *Eimeria maxima, Eimeria mitis, Eimeria tenella, Eimeria acervulina, Eimeria brunetti, Eimeria necatrix, Eimeria praecox,* and mixtures thereof. In one embodiment, separation of the oocysts is accomplished by a sodium sulfate flotation process which includes preparing an admixture comprising the oocysts and the sodium sulfate, centrifuging the slurry and recovering a supernatant therefrom, forming a dilution of the supernatant and centrifuging the dilution, and recovering the concentrate from the centrifuged dilution.

In another embodiment, separation the oocysts is accomplished by a gas flotation process which includes adjusting the first suspension to a pH sufficient to affect adhesion between bubbles of the gas in the suspension and the encysted protozoa, conditioning the pH adjusted suspension by adding a sufficient amount of a surface active agent compound to selectively coat particles in the suspension and a sufficient amount of a heteropolar compound to produce a stable froth, passing the conditioned suspension through at least one gas flotation cell, and recovering the encysted protozoa from the gas flotation cell. This embodiment may further include adding a bleaching agent to the sporulated oocysts in an amount sufficient to inactivate residual microorganisms and eliminate residual organic matter, and bleaching the sporulated oocysts. In another embodiment, the bleaching is conducted concurrently with the sporulation, and the bleaching agent is hydrogen peroxide. In yet another embodiment, the bleaching agent is sodium hypochlorite present in an amount from about 5,000 to about 10,000 parts per million free available chlorine, ozone present in an amount up to about 3% in air, and combinations thereof.

This method may further include washing the bleached oocysts by cross-flow membrane filtration to decrease the residual bleaching agent concentration to an acceptable level. In yet another embodiment, the bleaching agent is sodium hypochlorite present after washing in a concentration sufficient to suppress residual microbial growth, and the bleached and washed oocyst suspension has a concentration from about 1 x 10⁶ to about 2.5 x 10⁶ oocysts/ml, a maximum solids size of less than about 200 microns, and sodium sulfate present in an amount sufficiently low to not interfere with the viability and infectivity of the cysts or oocysts, preferably less that about 3 percent, more preferably less than about 0.9 percent This method may further include concentrating the bleached and washed oocysts into a sterile concentrate, combining sterile concentrates of one or more species of oocysts into a combined concentrate, and packaging the combined concentrate under sterile conditions.

### Brief Description Of The Drawings

Fig. 1A is a flow chart illustrating embodiments of the method of the present invention;
Fig. 1B is a flow chart illustrating embodiments of the method of the present invention;
Fig. 2 is a plot showing oocyst concentration verses sodium sulfate concentration; and
Fig. 3 is a plot showing sporulation percent verses time after cyst harvest.

### Detailed Description Of The Invention

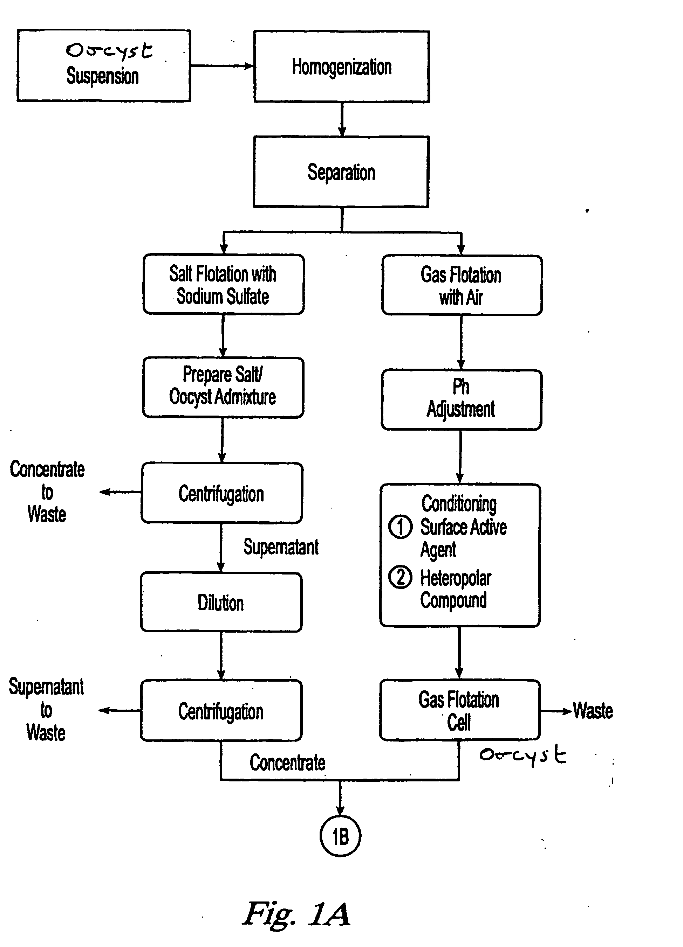

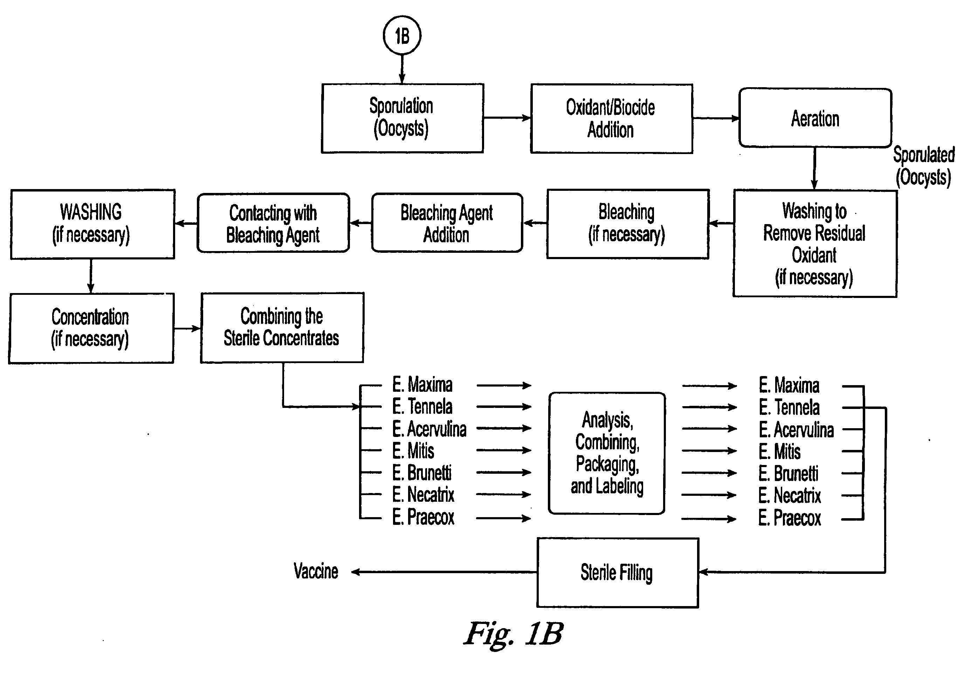

The present method is directed to producing a concentrate of sporulated oocysts to be utilized in a vaccine. Specifically, the method is directed to producing a concentrate of sporulated avian coccidial oocysts to be used to produce an in-ovo vaccine against coccidiosis. The production of a suitable and acceptable vaccine requires a multi-step treatment process constituting a plurality of unit processes. As shown in Figs. 1A and 1B, the vaccine production process can include taking a suspension of oocysts, homogenizing the suspension, separating the oocysts, from the suspension by either salt flotation or gas flotation, sporulating the oocysts, bleaching the sporulated oocysts, washing the bleached product, concentrating the oocysts, combining the concentrates from multiple species of oocysts, and sterile filling to produce a vaccine. These steps are described in further detail below. Oocysts, may be obtained from various sources including purified suspensions, intestinal linings, and fecal suspensions. Especially when the oocysts are obtained from feces, the suspensions or slurries can include significant amounts of undesirable suspended solids. For example, the suspensions can include from about 1 up to about 20 weight percent solids or feces. Preferably, the suspension includes from about 5 to about 20 weight percent solids when salt flotation is used and about 1 weight percent when gas flotation is used. In order to liberate the oocysts from the associated organics in the suspension, the suspension may be subjected to a homogenization step. The suspension may be homogenized for a time period from about greater than 0 to about 5 minutes, preferably 1 minute depending upon the intensity of the homogenization. The homogenization conditions should be sufficiently intense to liberate the oocysts but gentle enough to prevent their destruction.

The oocysts need to be separated from this first suspension, preferably to achieve at least about 70% encysted protozoa recovery and at least about 80% solids rejection. Typically, this results in an oocyst, concentration of about 1x10⁴ to about 1.5x10⁶ oocysts/ml. Separation can be achieved by either a salt flotation process or a gas flotation process.

When a salt flotation process is used, an admixture of the unpurified oocysts and the salt is prepared. Suitable salt solutions include dense solutions of water soluble salts including chlorides, sulfates, phosphates, nitrates, and acetates of ammonium, sodium, potassium, calcium, magnesium, and zinc. Suitable highly hydrogen-bonded organics including urea and the salts of guanidiene. Preferably, the salts include sodium sulfate, magnesium sulfate, magnesium chloride, calcium chloride, and mixtures thereof. More preferably, the salt is sodium sulfate. These salts provide the benefit of being significantly less corrosive to the process equipment. The salt is added in an amount sufficient to produce a difference between the specific gravities of the oocysts and the admixture. Preferably, the specific gravity of the oocysts will be less than the specific gravity of the admixture. The salt is present in an amount from about 3 to about 30 weight percent, preferably about 14 to about 20 weight percent, more preferably about 20 weight percent.

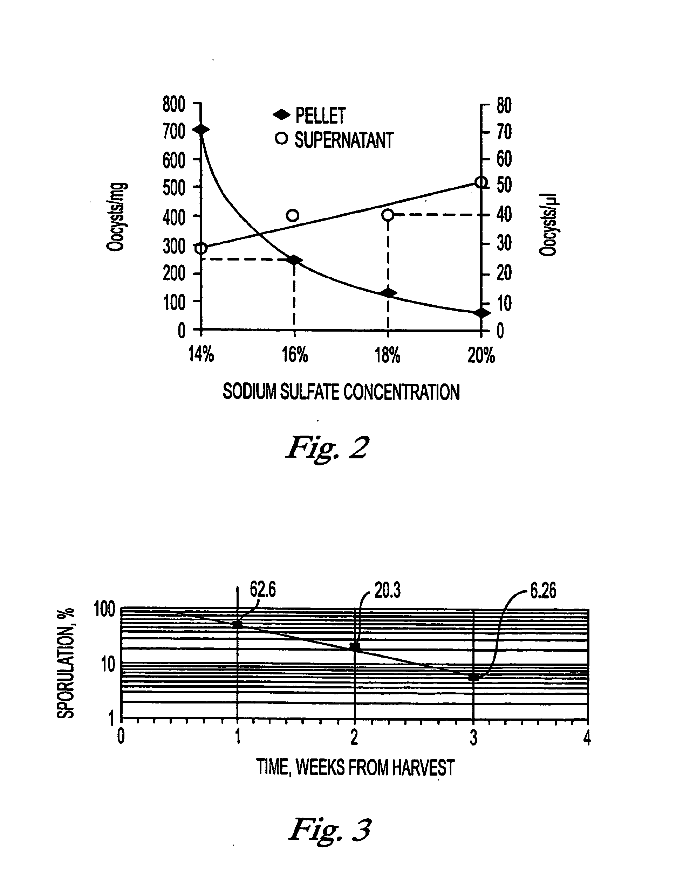

The admixture is then subject to centrifugation and the supernatant and concentrate are collected. Depending upon the admixture conditions, the oocysts can be contained in the supernatant or the concentrate. The admixture is centrifuged at about 3000 to about 15,000g, preferably 12,000g for a period of time necessary to adequately separate the oocysts typically up to about 10 minutes. For a 20% sodium sulfate solution, the oocysts will be contained in the supernatant: therefore, the supernatant is collected and the concentrate or pellet is directed to waste. As shown in Fig. 2, a sodium sulfate concentration of 20% provides for a high concentration of oocysts in the supernatant (product) and only a small amount of oocysts in the pellet (waste). A dilution is formed by the addition of water to the recovered supernatant. The supernatant is diluted sufficiently so that the specific gravity of the oocysts is more than the specific gravity of the dilution. Therefore, the oocysts will tend to settle in the dilution. For a sodium sulfate concentration of 20%, the ratio of the weight of the dilution to the weight of the supernatant is from about 4 to about 8.

The dilution is then centrifuged for a second time under comparable centrifugation conditions as in the first centrifugation, such that the residual salt concentration is less than about 10 weight percent, preferably less than about 1 weight percent, and the resulting cyst concentration is about 10⁴ to 10⁶ cysts/ml representing about 80 to about 95 percent recovery. The solid debris rejection rate is preferably about 90 to about 99 percent. In this embodiment, the oocysts are recovered from the concentrate and the supernatant is directed to waste.

In another embodiment, separation of the oocysts is accomplished by a gas flotation process. Preferably the gas is air, although any gas including oxygen and nitrogen could be used. When gas flotation is used, the pH of the suspension is adjusted to a pH sufficient to affect adhesion between the gas bubbles in the suspension and the oocysts by changing the surface chemistry of the oocysts. The pH is typically adjusted to a pH of about 2 to about 9, preferably about 2.5 to 3.5, more preferably about 3.

The pH adjusted suspension is additionally conditioned by the addition of a surface active agent compound, sometimes referred to as a collector compound and a heteropolar compound, sometimes referred to as a frother compound. The surface active agent compound, also termed a selective detergent, wetting agent, or emulsifier, is added in an amount sufficient to promote contact between selected solids and the gas bubbles by forming a thin coating over the particles to be floated, rendering these particles hydrophobic, while not coating other particles. Therefore, through selection of the surface active agent compound, the particles to be floated can be determined. Therefore, the oocysts could be collected in a gas flotation process in which they are floated or in a reverse flotation process in which the solids to be removed are floated. Suitable surface active agents include a sodium salt of long-chain alkyl hydrogen sulfate, a quaternary ammonium compound, a blend of a fatty ammonium acetate and 2-ethylhexanol, an ester/amide compound, an alkyloxy polyethylenoxyethanol, and mixtures thereof. Preferably, the surface active agent compound is sodium lauryl sulfate. The surface active agent should be present In an amount sufficient to coat the particles to be floated. The surface active agent is present in an amount from about 0.5 to 2 lb per ton of solids in the conditioned suspension, preferably 0.5 lb per ton of solids. Alternatively, the surface active agent compound can be about 0.5 lb dodecyl amine per ton of solids which has been acidified with hydrochloric acid until a neutral pH is obtained, or about 0.5 lb potassium salt of oleic acid per ton of solids.

The heteropolar compounds are selected for their ability to change the surface tension of the water and produce stable froths. Typically, these heteropolar compounds contain one or more hydrocarbon groups attached to one polar group, with the hydrocarbon radical having upwards of 5 or 6 carbon atoms. Suitable heteropolar compounds include amyl and butyl alcohols, terpinols, cresols, and mixtures thereof. Preferably, the heteropolar compound is methyl isobutyl carbinol (MIBC) also referred to as methyl amyl alcohol and most accurately referred to as 4-methylpentanol,-2. The heteropolar compound is present in an amount sufficient to produce a stable froth. Preferably, the heteropolar compound is present in an amount up to about 2.0 lb per ton of solids, more preferably from about 0.5 to 2.0 lb per ton of solids, most preferably about 0.5 lb per ton of solids. One ton equals 2240 lb.

The conditioned suspension is passed through a gas flotation cell and the oocysts are recovered. Depending upon the pH, surface active agent compounds, and heteropolar compound selected, the oocysts may be floated or retained in suspension. Preferably, the oocysts are floated. The gas flotation process is conducted for a period of time and at an air flow rate sufficient to recover about 20 to 100 percent, typically about 85 percent of the oocysts while rejecting about 20 to 90 percent, typically 70 percent of the solid debris. The gas flotation process is conducted for a period of time of about 3 minutes to greater than about 10 minutes, preferably about 10 minutes. The gas flow rate in the air flotation cell is about 0.25 to about 1.1 volumes of gas per volume of solution per minute ("vvm"), preferably about 0.25 to about 0.83 vvm, more preferably about 0.25 to about 0.75 wm. The gas flotation cell may include a plurality of serial flotation units. Preferably, the gas flotation cell includes at least two serial flotation units. Preferably, the flotation units are gas flotation columns. The gas flow rate in the serial flotation units may be the same or different. Preferably the gas flows are different, for example about 0.47 vvm for a 81.3 cm (32 inch) high, 5.08 cm (2 inch) diameter column and about 0.27 vvm for a 152.4 cm (60 inch) high, 5.08 (2 inch) diameter column.

The oocysts are sporulated. As is shown in Fig. 3, sporulation should occur as soon after harvesting the oocysts as possible, preferably, the oocysts should be sporulated within 3 days after harvest. An aqueous suspension of the oocysts and an oxidant or biocide is prepared, and the aqueous suspension is aerated for a period of time sufficient to sporulate the oocysts. If a salt flotation process was used to separate the oocysts, then the salt concentration during sporulation should be less than about 10 weight percent, preferably less than about 2 weight percent, more preferably less than about 1 weight percent. Preferably, the cyst concentration during sporulation is about 10⁴ to about 10⁶ oocysts/ml, more preferably about 10⁵ oocysts/ml. The oocysts should be sporulated within about 1 to 24 hours after recovery of the purified cyst concentrate. The pH of the aqueous suspension should be about 5.0 to 7.0, preferably about 5.2 to 6.8. The temperature of the aqueous suspension in one embodiment is about 20 to about 33 °C, preferably about 22 to about 32°C, more preferably about 25 to about 29°C, most preferably about 25°C. The aeration rate in one embodiment is about 0.1 to about 10 wm, preferably about 0.1 to about 2.0 wm. Overall, the agitation level should be sufficient to fully suspend all solids during sporulation but not enough to destroy the oocysts. This may occur through aeration, shaking, stirring, and combinations thereof. When stirring is used, for example, the stirring should be sufficient to keep the suspended solids in suspension but not enough to destroy the oocysts. Suitable stirring in a 15.2 cm (6 inch) diameter vessel can occur at about 121 to 204 rpm, preferably about 197 to 200 rpm, more preferably about 200 rpm. The dissolved oxygen content should be maintained at a level sufficient to permit the oocysts to sporulate, preferably about 80% of the saturation concentration at the given temperature. The time period for sporulation can be up to about 72 hours, preferably between about 40 and about 72 hours, more preferably about 48 to about 50 hours.

The oxidant is added in a sufficient amount to inactivate the undesirable microbial growth in the aqueous suspension. The oxidant or biocide is hydrogen peroxide.

In a preferred embodiment the oxidant is hydrogen peroxide present in an amount from about 1,000 to about 20,000 mg/l, preferably about 5,000 mg/l. Hydrogen peroxide provides the benefit of easier and less expensive handling, and the generation of a hazardous waste by-product from this process is alleviated.

Aeration can be accomplished by either shaking on a shaker table or air sparging in a sporulation tank (e.g. fermentation tank). Preferably, aeration is accomplished in an air sparging tank because the mass transfer of air is greater, thus reducing the time for sporulation and the required size of the equipment.

Following sporulation, if necessary, the oocysts may be washed to reduce the residual oxidant concentration to an acceptable level. Hydrogen peroxide provides the benefit of eliminating the need for this step. Washing may be accomplished by serial washings, preferably, washing is accomplished by membrane filtration, more preferably by diafiltration. In the case of membrane filtration, the membrane pore size is selected to allow passage of solutes through the membrane while restricting the passage of the oocysts from one side of the membrane to the other. In one embodiment, washing is conducted with water at a transmembrane pressure of about up to about 206 kPa (30 psi) preferably 138 kPa to 172 kPa (20 to 25 psi), a crossflow velocity of up to about 10 m/s, preferably about 2 m/s, and a flux through the membrane of up to about 10 l/min/m², preferably about 3 l/min/m².

The oocysts may be bleached to inactivate residual microorganisms and to eliminate residual organic matter. First, a sufficient amount of a bleaching agent is added, and then the oocysts are bleached or contacted with the bleaching agent for a sufficient period of time. The oocysts may be bleached for a period of up to 1.5 hours. Suitable bleaching agents include sodium hypochlorite, hydrogen peroxide, ozone, and mixtures thereof. The bleaching agent can be initially present in an amount of about 2,000 to about 20,000 mg/l, preferably about 8000 to about 10,000 mg/l, more preferably, 8000 mg/l. The bleaching agent should not be present in an amount sufficient to cause corrosion in the process equipment. When hydrogen peroxide is used for sporulation, bleaching and sporulation may be conducted concurrently. Following bleaching, the bleached suspension is washed, if necessary, to reduce the residual oxidant concentration to an acceptable level. When sodium hypochlorite is the bleaching agent, the acceptable level is less than about 1 mg/l. Washing can be accomplished by either serial washing or diafiltration.

The bleached suspension can be concentrated into a sterile concentration having a concentration high enough for efficient and effective handling. For example, the final concentrated oocyte suspension can include a maximum solids size of less that about 200 microns, preferably less than about 25 microns, a salt content of less than about 0.9 percent, a free residual chlorine concentration of less than about 1 mg/l but sufficient to keep residual microbial growth suppressed, and a cyst concentration of about 1 x 10⁶ to 2.5 x 10⁶ cysts/ml.

When numerous encysted protozoa or different species of one genus of encysted protozoa are to be used in a single vaccine, the sterile concentrates from the different species are combined into a single sterile concentrate. For example, the encysted protozoa can be avian coccidial oocysts including *Eimeria maxima, Eimeria mitis, Eimeria tenella, Eimeria acervulina, Eimeria brunetti, Eimeria necatrix, Eimeria praecox,* and mixtures thereof including multiple strains of each. Finally the combined concentrates are subject to filling and packaging under sterile conditions, and a vaccine is produced.

Although preferred embodiments of the present invention have been described, it is understood that the present method can be used to produce other vaccines for numerous types of animal and for humans. This method could, in fact, be used to immunize humans against typical waterborne protozoa such as *Cryptosporidium* and *Giardia lamblia.* Moreover, even though the entire recovery process is described, unit processes thereof may be used independently or inserted into other recovery, monitoring, or treatment schemes without departing from the scope and spirit of the present invention.

## Claims

1. A method for the sporulation of oocysts, comprising:
forming an aqueous suspension of the oocysts with water and hydrogen peroxide, wherein the hydrogen peroxide is present in an amount sufficient to eliminate unwanted microbiological growth; and
aerating the aqueous suspension to maintain the dissolved oxygen content at a level sufficient to permit the oocysts to sporulate, to thereby sporulate the oocysts.

2. The method of claim 1, wherein the oocysts are avian coccidial oocysts.

3. The method of claim 2, wherein the avian coccidial oocysts are selected from the group consisting of oocysts from *Eimeria maxima, Eimeria mitis, Eimeria tenella, Eimeria acervulina, Eimeria brunetti, Eimeria necatrix, Eimeria praecox,* and combinations thereof.

4. The method of any of claims 1-3, wherein the aqueous suspension is aerated for a time period up to about 72 hours.

5. The method of any of claims 1-4, wherein the aqueous suspension during aeration has a dissolved oxygen level greater than about 80% of the saturation level at a temperature of about 22°C to about 32°C and with an agitation level sufficient to adequately suspend all the solids.

6. The method of any one of preceding claims 4-5, wherein the aqueous suspension is aerated for a time period between about 40 and 72 hours.

7. The method of any of claims 1-6, wherein the aqueous suspension comprises an oocyst concentration of about 10⁴ to about 10⁶ oocysts/ml and an initial hydrogen peroxide concentration of about 1,000 to about 20,000 mg/l.

8. A method for the purification, recovery and sporulation of oocysts, comprising:
separating the oocysts from a first suspension comprising the oocysts; and
sporulating the separated oocysts by the method of any of claims 1-7.

9. The method of claim 8, wherein separating the oocysts is accomplished by a salt flotation process.

10. The method of claim 9, wherein the salt flotation process comprises:
preparing an admixture comprising the oocysts and the salt;
centrifuging the slurry and recovering a supernatant therefrom;
forming a dilution of the supernatant and centrifuging the dilution; and
recovering the concentrate from the centrifuged dilution.

11. The method of claim 9 or claim 10, wherein the salt comprises a salt selected from the group consisting of sodium sulfate, potassium sulfate, magnesium sulfate, sodium phosphate, potassium phosphate, magnesium phosphate, sodium acetate, potassium acetate, magnesium acetate, and mixtures thereof.

12. The method of claim 11, wherein the salt comprises sodium sulfate.

13. The method of claim 11, wherein the salt comprises magnesium sulfate.

14. The method of claim 8, wherein separating the oocysts is accomplished by a gas flotation process which comprises:
adjusting the first suspension to a pH sufficient to affect adhesion between bubbles of the gas in the suspension and the encysted protozoa;
conditioning the pH adjusted suspension by adding a sufficient amount of a surface active agent compound to selectively coat particles in the suspension and a sufficient amount of a heteropolar compound to produce a stable froth;
passing the conditioned suspension through at least one gas flotation cell; and
recovering the encysted protozoa from the gas flotation cell.

15. The method of claim 14, further comprising:
adding a bleaching agent to the sporulated oocysts in an amount sufficient to inactivate residual microorganisms and eliminate residual organic matter; and
bleaching the sporulated oocysts.

16. The method of claim 15, wherein the bleaching agent is hydrogen peroxide, sodium hypochlorite, ozone, or a mixture thereof.

17. The method of claim 16, wherein the bleaching agent is hydrogen peroxide and bleaching is conducted concurrently with sporulation.

18. The method of claim 16, wherein the bleaching agent is:
(a) sodium hypochlorite in an amount from about 5,000 to about 10,000 parts per million free available chlorine,
(b) ozone in an amount up to about 3% in air,
(c) hydrogen peroxide in an amount from about 2,000 to about 20,000 mg/l, or
(d) any combination of (a), (b) and (c).

19. The method of any of claims 15-18, further comprising washing the bleached oocysts by cross-flow membrane filtration to decrease the residual bleaching agent concentration to an acceptable level.

20. The method of claim 19, wherein the bleached and washed oocyst suspension has a concentration from about 1 x 10⁶ to about 2.5 x 10⁶ oocysts/ml, a maximum solids size of less than about 200 microns, and a salt content of less than about 0.9 percent.

21. The method of claim 19 or claim 20, further comprising:
concentrating the bleached and washed oocysts into a sterile concentrate;
combining sterile concentrates of one or more species of oocysts into a combined concentrate; and
packaging the combined concentrate under sterile conditions.

## Patentansprüche

1. Verfahren für die Sporulation von Oozysten, umfassend:
Erzeugen einer wässrigen Suspension der Oozysten mit Wasser und Wasserstoffperoxid, wobei das Wasserstoffperoxid in einer ausreichenden Menge vorliegt, um unerwünschtes mikrobiologisches Wachstum zu eliminieren, sowie
Belüften der wässrigen Suspension, um den Gehalt an aufgelöstem Sauerstoff auf einem ausreichenden Wert zu halten, um den Oozysten die Bildung von Sporen zu ermöglichen, um dadurch die Sporulation der Oozysten herbeizuführen.

2. Verfahren nach Anspruch 1, bei welchem die Oozysten aviäre coccidiale Oozysten sind.

3. Verfahren nach Anspruch 2, wobei die aviären coccidialen Oozysten ausgewählt sind aus der Gruppe, bestehend aus Oozysten von: *Eimeria maxima, Eimeria mitis, Eimeria tenella, Eimeria acervulina, Eimeria brunetti, Eimeria necatrix, Eimeria praecox* und Kombinationen davon.

4. Verfahren nach einem der Ansprüche 1 bis 3, wobei die wässrige Suspension für eine Zeitdauer bis zu etwa 72 Stunden belüftet wird.

5. Verfahren nach einem der Ansprüche 1 bis 4, wobei die wässrige Suspension während der Belüftung einen Gehalt an aufgelöstem Sauerstoff größer als etwa 80% des Sättigungswertes bei einer Temperatur von etwa 22° bis etwa 32°C hat mit einem Bewegungsniveau, das ausreichend ist, um alle Feststoffe hinreichend zu suspendieren.

6. Verfahren nach einem der vorgenannten Ansprüche 4 bis 5, wobei die wässrige Suspension für eine Zeitdauer zwischen etwa 40 und 72 Stunden belüftet wird.

7. Verfahren nach einem der Ansprüche 1 bis 6, wobei die wässrige Suspension eine Oozysten-Konzentration von etwa 10⁴ bis etwa 10⁶ Oozysten/ml aufweist und eine anfängliche Konzentration an Wasserstoffperoxid von etwa 1.000 bis etwa 20.000 mg/l.

8. Verfahren zur Reinigung, Gewinnung und Sporulation von Oozysten, umfassend:
Abtrennen der Oozysten von einer ersten Suspension, welche die Oozysten aufweist; und
Sporulation der abgetrennten Oozysten mit Hilfe des Verfahrens nach einem der Ansprüche 1 bis 7.

9. Verfahren nach Anspruch 8, wobei das Abtrennen der Oozysten mit Hilfe eines Salz-Flotationsprozesses erzielt wird.

10. Verfahren nach Anspruch 9, wobei der Salz-Flotationsprozess umfasst:
Herstellen einer Zumischung, welchen die Oozysten und Salz aufweist;
Zentrifugieren der Aufschlämmung und Gewinnen eines Überstandes daraus;
Erzeugen einer Verdünnung des Überstandes und Zentrifugieren der Verdünnung; und
Gewinnen des Konzentrats aus der zentrifugierten Verdünnung.

11. Verfahren nach Anspruch 9 oder 10, wobei das Salz ein Salz aufweist, das ausgewählt ist aus der Gruppe, bestehend aus: Natriumsulfat, Kaliumsulfat, Magnesiumsulfat, Natriumphosphat, Kaliumphosphat, Magnesiumphosphat, Natriumacetat, Kaliumacetat, Magnesiumacetat und Mischungen davon.

12. Verfahren nach Anspruch 11, wobei das Salz Natriumsulfat aufweist.

13. Verfahren nach Anspruch 11, wobei das Salz Magnesiumsulfat aufweist.

14. Verfahren nach Anspruch 8, wobei das Abtrennen der Oozysten mit Hilfe eines Prozesses der Gasflotation erzielt wird, das umfasst:
Einstellen der ersten Suspension auf einen ausreichenden pH-Wert, um eine Adhäsion zwischen den Gasbläschen in der Suspension und den enzystierten Protozoen herbeizuführen;
Konditionieren der pH-eingestellten Suspension durch Zugabe einer ausreichenden Menge einer Verbindung eines grenzflächenaktiven Mittels, um in der Suspension selektiv Teilchen zu beschichten, und einer ausreichenden Menge einer heteropolaren Verbindung, um einen stabilen Schaum zu erzeugen;
die konditionierte Suspension durch mindestens eine Gasflotationszelle leiten und
Gewinnen der enzystierten Protozoen aus der Gasflotationszelle.

15. Verfahren nach Anspruch 14, ferner umfassend:
Zugabe eines bleichenden Mittels zu den sporulierten Oozysten in einer ausreichenden Menge, um restliche Mikroorganismen zu inaktivieren und restliche organische Substanz zu eliminieren, und
Bleichen der sporulierten Oozysten.

16. Verfahren nach Anspruch 15, wobei das bleichende Mittel Wasserstoffperoxid ist, Natriumhypochlorit, Ozon oder eine Mischung davon.

17. Verfahren nach Anspruch 16, wobei das bleichende Mittel Wasserstoffperoxid ist und des Bleichen gleichzeitig mit der Sporulation ausgeführt wird.

18. Verfahren nach Anspruch 16, wobei das bleichende Mittel ist:
(a) Natriumhypochlorit in einer Menge von etwa 5.000 bis etwa 10.000 Teilen pro Million frei verfügbares Chlor,
(b) Ozon in einer Menge bis zu etwa 3% in Luft,
(c) Wasserstoffperoxid in einer Menge von etwa 2.000 bis etwa 20.000 mg/l oder
(d) eine beliebige Kombination von (a), (b) und (c).

19. Verfahren nach einem der Ansprüche 15 bis 18, ferner umfassend das Waschen der gebleichten Oozysten durch Querstrom-Membranfiltration, um die restliche Konzentration des bleichenden Mittels bis auf einen akzeptablen Wert herabzusetzen.

20. Verfahren nach Anspruch 19, wobei die gebleichte und gewaschene Oozysten-Suspension eine Konzentration von etwa 1 x 10⁶ bis etwa 2,5 x 10⁶ Oozysten pro Milliliter hat, eine maximale Partikelgröße der Feststoffe von weniger als etwa 200 Mikrometer und einen Salzgehalt von weniger als etwa 0,9 Prozent.

21. Verfahren nach Anspruch 19 oder 20, ferner umfassend:
Einengen der gebleichten und gewaschenen Oozysten zu einem sterilen Konzentrat;
Vereinen steriler Konzentrate einer oder mehrerer Spezies von Oozysten zu einem vereinten Konzentrat und
Verpacken des vereinten Konzentrats unter sterilen Bedingungen.

## Revendications

1. Procédé pour la sporulation d'ookystes, comprenant :
la formation d'une suspension aqueuse des ookystes avec de l'eau et du peroxyde d'hydrogène, où le peroxyde d'hydrogène est présent en une quantité suffisante pour éliminer la croissance microbiologique indésirable ; et
l'aération de la suspension aqueuse pour maintenir la teneur en oxygène dissous à un niveau suffisant pour permettre aux ookystes de sporuler, pour faire ainsi sporuler les ookystes.

2. Procédé selon la revendication 1, où les ookystes sont des ookystes coccidiens aviaires.

3. Procédé selon la revendication 2, où les ookystes coccidiens aviaires sont choisis dans le groupe consistant en les ookystes de *Eimeria maxima, Eimeria mitis, Eimeria tenella, Eimeria acervulina, Eimeria brunetti, Eimeria necatrix, Eimeria praecox,* et leurs combinaisons.

4. Procédé selon l'une quelconque des revendications 1-3, où la suspension aqueuse est aérée pendant une durée pouvant atteindre environ 72 heures.

5. Procédé selon l'une quelconque des revendications 1-4, où la suspension aqueuse pendant l'aération a un niveau d'oxygène dissous supérieur à environ 80% du niveau de saturation à une température d'environ 22°C à environ 32°C et avec un niveau d'agitation suffisant pour mettre adéquatement en suspension tous les solides.

6. Procédé selon l'une quelconque des revendications 4-5 précédentes, où la suspension aqueuse est aérée pendant une durée entre environ 40 et 72 heures.

7. Procédé selon l'une quelconque des revendications 1-6, où la suspension aqueuse comprend une concentration d'ookystes d'environ 10⁴ à environ 10⁶ ookystes/ml et une concentration initiale de peroxyde d'hydrogène d'environ 1000 à environ 20000 mg/l.

8. Procédé pour la purification, la récupération et la sporulation d'ookystes, comprenant:
la séparation des ookystes d'avec une première suspension comprenant les ookystes ; et
la sporulation des ookystes séparés par le procédé selon l'une quelconque des revendications 1-7.

9. Procédé selon la revendication 8, où la séparation des ookystes est accomplie par un procédé de flottation avec un sel.

10. Procédé selon la revendication 9, où le procédé de flottation avec un sel comprend :
la préparation d'un mélange comprenant les ookystes et le sel ;
la centrifugation de la suspension et la récupération d'un surnageant à partir de celle-ci ;
la formation d'une dilution du surnageant et la centrifugation de la dilution ; et
la récupération du concentré à partir de la dilution centrifugée.

11. Procédé selon la revendication 9 ou la revendication 10, où le sel comprend un sel choisi dans le groupe consistant en le sulfate de sodium, le sulfate de potassium, le sulfate de magnésium, le phosphate de sodium, le phosphate de potassium, le phosphate de magnésium, l'acétate de sodium, l'acétate de potassium, l'acétate de magnésium, et leurs mélanges.

12. Procédé selon la revendication 11 où le sel comprend du sulfate de sodium.

13. Procédé selon la revendication 11, où le sel comprend du sulfate de magnésium.

14. Procédé selon la revendication 8, où la séparation des ookystes est accomplie par un procédé de flottation avec un gaz qui comprend :
l'ajustement de la première suspension à un pH suffisant pour affecter l'adhésion entre les bulles du gaz dans la suspension et les protozoaires enkystés ;
le conditionnement de la suspension à pH ajusté par addition d'une quantité suffisante d'un composé agent tensioactif pour revêtir sélectivement les particules dans la suspension et d'une quantité suffisante d'un composé hétéropolaire pour produire une écume stable ;
le passage de la suspension conditionnée dans au moins une cellule de flottation avec un gaz ; et
la récupération des protozoaires enkystés à partir de la cellule de flottation avec un gaz.

15. Procédé selon la revendication 14, comprenant en outre :
l'addition d'un agent de blanchiment aux ookystes sporulés en une quantité suffisante pour inactiver les microorganismes résiduels et éliminer la matière organique résiduelle ; et
le blanchiment des ookystes sporulés.

16. Procédé selon la revendication 15, où l'agent de blanchiment est le peroxyde d'hydrogène, l'hypochlorite de sodium, l'ozone, ou un mélange de ceux-ci.

17. Procédé selon la revendication 16, où l'agent de blanchiment est le peroxyde d'hydrogène et le blanchiment est accompli en même temps que la sporulation.

18. Procédé selon la revendication 16, où l'agent de blanchiment est :
(a) l'hypochlorite de sodium en une quantité d'environ 5000 à environ 10000 parties par million de chlore disponible libre,
(b) l'ozone en une quantité pouvant atteindre environ 3% dans l'air,
(c) le peroxyde d'hydrogène en une quantité d'environ 2000 à environ 20000 mg/l, ou
(d) toute combinaison de (a), (b) et (c).

19. Procédé selon l'une quelconque des revendications 15-18, comprenant en outre le lavage des ookystes blanchis par filtration sur membrane à courant transversal pour faire diminuer la concentration résiduelle de l'agent de blanchiment jusqu'à un niveau acceptable.

20. Procédé selon la revendication 19, où la suspension d'ookystes blanchis et lavés a une concentration d'environ 1x10⁶ à environ 2,5x10⁶ ookystes/ml, une taille maximale des solides inférieure à environ 200 µm, et une teneur en sel inférieure à environ 0,9 pourcent.

21. Procédé selon la revendication 19 ou la revendication 20, comprenant en outre :
la concentration des ookystes blanchis et lavés en un concentré stérile ;
la combinaison des concentrés stériles d'une ou plusieurs espèces d'ookystes en un concentré combiné ; et
l'emballage du concentré combiné dans des conditions stériles.
